# EUROPÄISCHE PATENTSCHRIFT

(11) **EP 0 485 874 B1**
(45) Veröffentlichungstag und Bekanntmachung des Hinweises auf die Patenterteilung: **20.09.1995**
(21) Anmeldenummer: 91118879.5
(22) Anmeldetag: 06.11.1991
(51) Int. Cl.: G01N 33/553, G01N 33/543, G01N 33/547

(54) **Immunsensorischer Wandler und Verfahren zu seiner Herstellung**
Transducer for immunosensor and its method of production
Transducteur pour immunosenseur et méthode de sa production

(30) Priorität: 14.11.1990 CH 3606/90
(43) Veröffentlichungstag der Anmeldung: 20.05.1992
(73) Patentinhaber: F. HOFFMANN-LA ROCHE AG, 4002 Basel (CH)
(72) Erfinder: Barner, Richard, CH-4108 Witterswil (CH); Huber, Walter, CH-4303 Kaiseraugst (CH); Hübscher, Josef, CH-4208 Nunningen (CH); Schlatter, Daniel, CH-4104 Oberwil (CH)
(74) Vertreter: Buntz, Gerhard

(56) Entgegenhaltungen:
- EP-A- 0 396 116
- WO-A-90/05303
- US-A- 4 529 712
- LANGMUIR Bd. 5, Nr. 3, 1989, WASHINGTON DC Seiten 723 - 727 BAIN ET AL. 'Comparison of self-assembled monolayers on gold: coadsorption of thiols and disulfides'
- LANGMUIR Bd. 4, Nr. 2, 1988, WASHINGTON DC Seiten 365 - 385 TROUGHTON ET AL. 'Monolayer films prepared by the spontaneous self-assambly of .....'

## Beschreibung

Die Erfindung betrifft einen immunsensorischen Wandler, sowie ein Verfahren zu seiner Herstellung

Das Prinzip eines Immunoassays beruht auf dem selektiven Erkennen eines Liganden, z.B. eines Antigens (Ag) oder eines Haptens (Hap), durch einen Antiliganden, z.B. einen Antikörper (Ak). Bei den üblicherweise in der Praxis eingesetzten Immunoassays handelt es sich um sog. kompetitive Assays oder Sandwichassays. Beide Verfahren arbeiten mit markierten Liganden oder Antiliganden. Je nach Art des sog. Markers unterscheidet man zwischen Radioimmunoassay (RIA), Enzymimmunoassay (EIA), Fluoreszenzimmunoassay (FIA), etc.

So wird zum Beispiel beim Sandwichassay ein erster Antikörper gegen ein Antigen auf einer Festphase (Tube in der sog."coated tube"-Technik; Bead in der sog. "coated bead" Technik) immobilisiert. Probenantigen und ein markierter zweiter Antikörper werden miteinander und mit der Festphase inkubiert. Dabei werden Bindungsplätze an der Oberfläche, die durch den immobilisierten ersten Antikörper vorgebildet sind durch den Komplex zwischen Antigen und zweitem Antikörper besetzt. Die Bestimmung der Konzentration an Probenantigen erfolgt anschliessend über die Bestimmung der Labelkonzentration an der Oberfläche, d.h. beim EIA-Verfahren durch Inkubation mit dem Enzymsubstrat, beim FIA-Verfahren durch Messung der Fluoreszenzeigenschaften und beim RIA-Verfahren durch Messung der radioaktiven Strahlung. Alle oben aufgezählten Verfahren benötigen zeitaufwendige Wasch- und Inkubationsschritte.

In der neueren Literatur werden deshalb in zunehmendem Masse Messtechniken vorgeschlagen welche einen Nachweis des Ag-Ak-Komplexes ohne Zuhilfenahme eines Labels (Radioisotop, Enzym, Fluoreszenzfarbstoff) ermöglichen sollen.Die Verfahren werden unter dem Begriff Immunsensorik zusammengefasst. Die Immunsensoren sind im allgemeinen aufgebaut aus einem immunologisch erkennenden Element welches eng mit einem physikalischen Signalwandler verknüpft oder in diesen integriert ist. Die Aufgabe des immunologisch erkennenden Elementes ist das selektive Erkennen und Binden des interessierenden Analyten aus einer Analyselösung. Die damit einhergehende physikalische Veränderung des Elementes (z.B. Massenzunahme, Schichtdickenzunahme, Änderung der Dielektrizitätskonstante etc.) wird durch den Wandler erfasst und in ein Signal umgewandelt.

Bei den physikalischen Messverfahren welche diesen Wandlern zugrunde liegen, handelt es sich beispielsweise um die Oberflächenplasmonresonanz (Opt.Comm. 18, 395 - 399 (1976); Sensors and Actuators 3, 79-88 (1982/83)), die Gitterkopplung (Analyt.Lett. 19, 205-216 (1986); Sensors and Actuators 15, 273 - 284 (1988)), die Potentiometrie (J.Memb.Sci. 125-132 (1977); J.Anal.Chim.Acta 136, 93 - 99 (1982)), die Konduktometrie (Anal.Chem.Acta 60, 2374 - 2379 (1988)) oder die piezoelektrische Gravimetrie (Anal.Chem. 55, 2333 -2336 (1983)). Mit Ausnahme des Gitterkopplerverfahrens (dielektrische Wandleroberfläche) müssen bei allen anderen Messverfahren die immunologisch erkennenden Elemente auf eine metallische Wandleroberfläche aufgebracht werden.

Gold ist auf Grund seiner Korrosionsbeständigkeit und in der Oberflächenplasmonresonanz auf Grund seiner optischen Eigenschaften ein bevorzugtes Metall. Während die oben aufgelisteten Messtechnologien schon weit entwickelt und charakterisiert sind, fehlen Technologien, um die selektiv erkennenden Elemente in geeigneter Form auf die entsprechenden Wandleroberflächen aufzubringen. Da bei all diesen Verfahren kein Label eingesetzt wird und die dadurch möglichen Verstärkungseffekte wegfallen, werden an diese Elemente sehr hohe Ansprüche gestellt. Die wichtigsten Anforderungen sind eine hohe Aktivität und Selektivität bezüglich des Bindens des nachzuweisenden Analyten. Diese beiden Anforderungen sind durch die bei der "coated tube" und "coated bead" - Technologie verwendeten physikalischen Adsorption der immunologischen Komponenten (z.B. Ak, Ag) zuwenig erfüllt. Diese Adsorption, welche bevorzugt auf hydrophoben Oberflächen erfolgt, hat folgende gravierenden Nachteile:
i) Die Proteine liegen nach der Adsorption in beliebiger Orientierung auf der Oberfläche. Dadurch werden zum Teil die für die Komplexbildung aktiven Domänen (Epitope, Antigen-Bindungsstellen) einzelner Proteine maskiert.
ii) Durch eine starke hydrophobe Wechselwirkung werden Proteine an einer derartigen Oberfläche denaturiert. Dadurch ist die Selektivität der Oberfläche für nur einen Analyten nicht mehr gewährleistet.
iii) Eine nicht vollständige Belegung der Oberfläche durch Adsorption führt dazu, dass auf Grund der adsorptiven Kräfte der Oberfläche auch Begleitproteine an die Oberfläche adsorbieren. Durch diese unspezifische Adsorption werden die Testergebnisse sowohl bei einem indirekten als auch bei einem direkten Nachweis erheblich verfälscht.

Während Verfahren für die geeignete Immobilisierung von Antiliganden auf Kunststoff- oder auf Glasoberflächen beispielsweise aus der Affinitätschromatographie hinlänglich bekannt sind, ist die gezielte Immobilisierung von Proteinmolekülen auf eine Goldoberfläche erst neuerdings bekannt geworden (vgl.WO 90/05303). In dieser Patentpublikation wird die Notwendigkeit einer dreidimensionalen Matrix (bevorzugt eine Polysaccharidmatrix z.B. bestehend aus Dextran) zur Erreichung einer hohen Sensitivität und eines ausreichenden dynamischen Bereiches besonders hervorgehoben. Das Verfahren beruht auf der Immobilisierung von hochmolekularen Polysacchariden über Thioundekanderivate, wobei letztere für die kovalente Verankerung des Polysaccharides auf der Goldoberfläche verantwortlich sind. Die Herstellung einer dreidimensionalen Matrix ist aber so aufwendig, dass dadurch die Verwirklichung eines nach einmaligem Gebrauch wegwerfbaren Sensors in Frage gestellt ist.

Der vorliegenden Erfindung liegt daher die Aufgabe zugrunde, einen immunsensorischen Wandler mit hoher Aktivität und Selektivität in Bezug auf das Binden des Analyten auf einer Matrix von Erkennungsmolekülen (Antikörper, Antigen) bereitzustellen.

Ueberraschenderweise wurde nun gefunden, dass zur Erreichung einer hohen Sensitivität und eines ausreichenden dynamischen Bereiches eine dreidimensionale Matrix nicht zwingend notwendig ist. Der Verzicht auf eine derartige dreidimensionale Matrix vereinfacht das Immobilisierungsverfahren beträchtlich.

Erfindungsgemäss wird die Aufgabe gelöst durch einen Wandler gemäß Anspruch 1, der aus einen Träger aus Gold, eine auf dessen Oberfläche angelagerte monomolekulare organische Zwischenschicht und einer auf dieser immobilisierten Liganden- oder Antiligandenschicht besteht.

Im erfindungsgemässen Verfahren gemäß Anspruch 5 wird auf einer Oberfläche eines metallischen Trägers durch spontane Chemisorption eine monomolekulare Schicht aus Alkylderivaten mit schwefelhaltigen Gruppen aufgebracht und anschliessend auf dieser Schicht ein für den gewünschten Immunnachweis geeigneter Ligand oder Antiligand immobilisiert.

Es wurde nämlich überraschend gefunden, dass mit diesem Verfahren beispielsweise Proteine auf Metalloberflächen, in geeigneter Art und Weise immobilisiert werden können. Die dabei entstehenden Oberflächen besitzen eine nachweisbar höhere Aktivität bezüglich der Ausbildung des Immunkomplexes an der Oberfläche als die bisher im Zusammenhang mit den oben erwähnten Messverfahren eingesetzten Oberflächen, wie beispielsweise die spontane Adsorption oder das Aufbringen einer dünnen Polymerschicht auf der Oberfläche durch Silanisierung oder Plasmapolymerisation und anschliessendes Immobilisieren nach bekannten Verfahren aus der Affinitätschromatographie.

Sollen auf einer Oberfläche Proteine in gezielter und gerichteter Art und Weise immobilisiert werden, so muss diese Oberfläche so vorbereitet werden, dass sie eine Bifunktionalität aufweist; d.h. die Oberfläche muss mit funktionellen Gruppen versehen werden, welche eine spontane Adsorption der Proteine verhindern, sowie mit funktionellen Gruppen, welche die kovalente Bindung mit der am Protein dafür vorgesehenen Gruppe eingehen.

Nach einer bevorzugten Ausführungsform des erfindungsgemässen Verfahrens werden die Goldoberflächen in einem ersten Schritt mit einer dünnen organischen Zusatzschicht versehen. Das Aufbringen dieser organischen Zusatzschichten beruht auf der an sich bekannten spontanen Chemisorption von Alkylderivaten mit schwefelhaltigen Gruppen (Thiole, Sulfide, Disulfide) aus Lösungen dieser Verbindungen an Gold unter Ausbildung von Monolagen [Langmuir 5, 723 -727 (1989); Langmuir 4, 365 - 385 (1988); J.Phys.Chem 91, 6663 (1987)].

Die Chemisorption erfolgt derart, dass die S-haltige Gruppe an der Goldoberfläche verankert ist und die Alkankette annähernd vertikal von dieser Oberfläche wegsteht. Die über diese Verfahren herstellbaren Schichtdicken können durch die Verwendung unterschiedlich langer Alkanketten (C₁₁-C₂₀) in einem Bereich von ca 1.3 - 3 nm variiert werden.

Die Ausbildung von Monolagen mit diesen Verbindungen erfolgt auch dann, wenn diese Verbindungen in ω-Stellung zur S-haltigen Gruppe mit funktionellen Gruppen, z.B. -COOH, -NH2, -OH etc. versehen sind. Es gelingt mit diesen Reagenzien also, eine Goldoberfläche mit einer dünnen organischen Schicht zu versehen, wobei diese organischen Zusatzschichten über eine Grenzfläche (Organische Schicht - umgebendes Medium) mit dicht gepackten funktionellen Gruppen verfügen. Dabei kann die Zusammensetzung der Oberfläche bezüglich der Konzentration an unterschiedlichen funktionellen Gruppen über die Konzentrationsverhältnisse der entsprechenden Reagenzien in der für die Adsorption verwendeten Lösung gesteuert werden. Mit diesem Verfahren können auf überraschend einfachem Wege Oberflächen für die Immobilisierung mit der geforderten Bifunktionalität hergestellt werden.

Es wurde ausserdem gefunden, dass eine spontane Adsorption von Proteinen überraschend stark zurückgedrängt wird, wenn die für den Aufbau der organischen Zusatzschichten verwendeten S-haltigen Reagenzien als zweite funktionelle Gruppe ein Hydroxyl, Polyhydroxyl, ein Ethylenglykol, ein Oligoethylenglykol, oder ein Monosaccharid als Strukturelement enthalten.

Zudem können die in diesen endständigen Gruppen vorkommenden Hydroxylgruppen in an sich bekannter Weise, z.B. durch Bromcyanaktivierung, Cyanurchloridaktivierung, Tosylchloridaktivierung, Epichlorhydrinaktivierung oder Carbonyldiimidazolaktivierung (Meth. in Enzymology 135, 30 - 65 (1987)) für das kovalente Binden von Proteinen verwendet werden. Obwohl bei diesen Verfahren z.Teil sehr aggressive Reagenzien verwendet werden müssen, bleiben bei diesen Aktivierungsschritten die einmal gebildeten Monolagen überraschenderweise intakt.

Das erfindungsgemässe Verfahren besteht darin, daß solche hydroxylhaltige Derivate, welche die spontane Adsorption der Proteine während einer Immobilisierung verhindern, gemeinsam mit Derivaten auf die Oberfläche gebracht werden, welche über spezielle Gruppen für das kovalente Binden von Proteinen verfügen. Es wird dadurch möglich, die geforderte Bifunktionalität einer Oberfläche auf einen beliebigen relativen Anteil der funktionellen Gruppen einzustellen. Dies ist bei der direkten Aktivierung hydroxylreicher Oberflächen nicht in vergleichbarem Ausmass möglich.

Die Reagenzien, welche über funktionelle Gruppen für das kovalente Binden verfügen, teilen sich in zwei Gruppen auf. Die erste Gruppe beinhaltet Derivate mit funktionellen Gruppen, welche erst nach einem zusätzlichen Aktivierungsschritt für das kovalente Binden verwendet werden können. Diese Aktivierung erfolgt nach der Chemisorption der Derivate auf der Goldoberfläche. Es sind Derivate welche neben den S-haltigen Strukturelementen eine Carbonsäurefunktion (Aktivierung durch Chlorameisensäureethylester [J.Am.Chem.Soc. 74, 676 - 678 (1952)] oder durch Umsetzen mit Carbodiimid, Hydroxysuccinimid oder Thionylchlorid [Meth.in Enzymology135, 30 - 65 (1987)], eine Amingruppe (Aktivierung durch Umsetzen mit Bernsteinsäureanhydrid und anschliessende Aktivierung der Carbonsäure [ebd.]), eine aromatische Amino- oder Nitrogruppe (Aktivierung durch Überführung in das Diazoniumsalz [ebd.]) oder eine Phenylazidogruppe (Photochemische Aktivierung [Biochemistry 16 5650 -5654 (1977); Biochemistry 17, 1403 -1408 (1978)]) enthalten. Auch hier wurde gefunden, dass die z.Teil drastischen Methoden der Aktivierung diese organischen Zusatzschichten auf den Goldoberflächen nicht zerstören.

Die zweite Gruppe umfasst S-haltige Alkylderivate, welche nach dem Aufbringen auf die Goldoberfläche keinen Aktivierungsschritt verlangen. Auch diese Reagenzien ordnen sich in Form von Monolagen auf der Metalloberfläche an. Sie besitzen funktionelle Gruppen, welche spezifisch mit einer Gruppe am Protein reagieren können. Beispiele für diese Gruppen sind Maleinimid (Reaktion mit -SH-Gruppe eines Fab'-Fragmentes, p-Benzoquinon, Cyanurchlorid, Epoxid, Sulfonsäurederivate, Carbonsäureazid (Reaktion mit NH₂-Funktion am Protein) oder Hydrazid (Reaktion mit Aldehydfunktionen an aufoxidierten Carbohydratresten). Diese Verbindungen sind jedoch päparativ nur herstellbar wenn die S-haltige Gruppierung nicht Thiol ist. Der Vorteil der Verbindungen aus der zweiten Gruppe besteht demnach darin, dass Goldoberflächen direkt mit einer für die kovalente Proteinimmobilisierung reaktionsfähigen Gruppen versehen werden können.

Das Immobilisierungsverfahren auf Goldoberflächen für Moleküle, welche in einem Immunnachweis verwendet werden können (z.B. Ak, Ag, Fab'-Fragmente, Haptene), basiert auf der Verwendung von Verbindungen der allgemeinen Formel

**R¹-(CH₂)**_{**n**}**-R²**

worin R¹ eine Gruppe S-X ist und X = H oder -(CH₂)ₘ-R²
oder -S-(CH₂)ₚ-R² bedeutet, wobei m und p = n sein können und n = 1-20 ist,
oder worin R¹ eine cyclische Disulfidgruppe der Formel 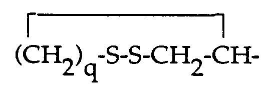
mit q = 1 oder 2 darstellt und n die obige Bedeutung hat und R² eine funktionelle Gruppe der Formel Y ist,
die gegebenenfalls durch Aktivierung in eine mit Protein koppelbare Gruppe Z umgewandelt werden kann, oder worin R² direkt obige Bedeutung von Z hat, wobei für R² = Z R¹ = SH (d.h die Verbindung ist schon aufgezogen als Thiolat und Y wird nach dem Aufziehen in Z umgewandelt, oder die Verbindung beinhaltet schon Z und R¹ = -S- oder -S-S-).

Typische Beispiele für Y sind:
-OH,
-CHOHCH₂CHOHCH₂OH,
-CHOHCH₂OH,
-(OCH₂CH₂)ₘOH (m=1-5),
-Monosaccharid,
-COOH,
-NH₂, 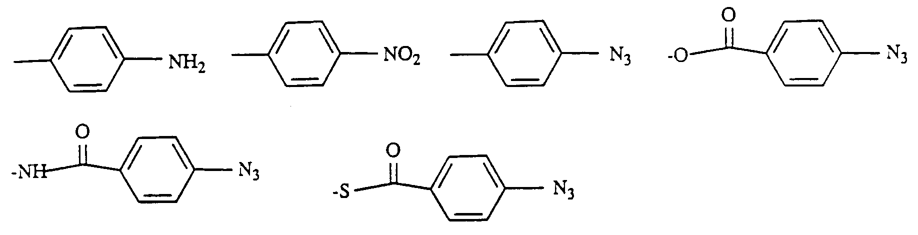
Typische Beispiele von Z sind:
a) Aktivierung von Alkoholen 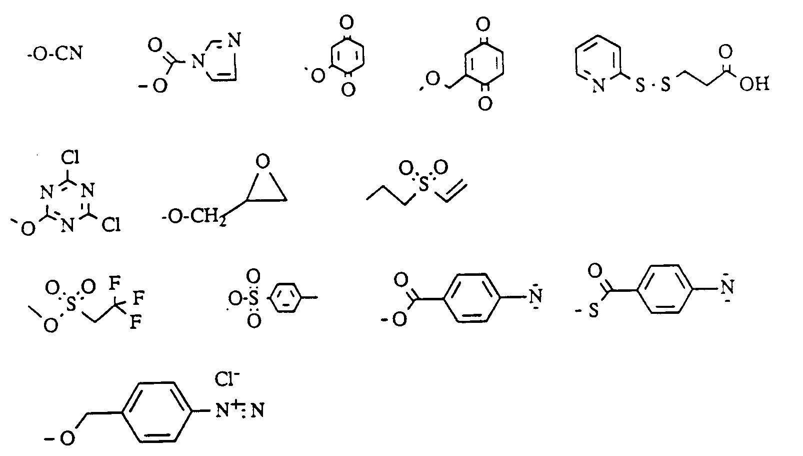
b) Aktivierung von Carbonsäuren 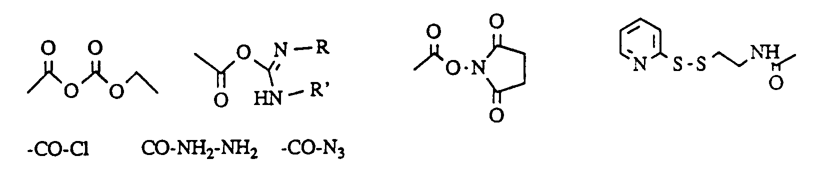
c) Aktivierung von Aminen

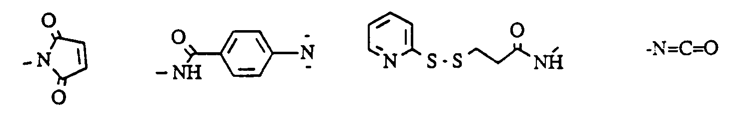

### Beispiele

Beispiel 1: 254 mg (0,5mMol) 22-Tosyloxi-1-docosanol wurden mit 200 mg (1,75mMol) Kaliumthioacetat in 20ml Ethanol während 2h am Rückfluss erwärmt. Dann wurden bei Raumtemp. 2ml 1n NaOH zugefügt und das Gemisch über Nacht gerührt. Nach Ansäuern mit 1n HCl wurde die Reaktionsmischung am Rollverdampfer eingeengt und der Rückstand an Kieselgel mit Toluol: EtOAc = 2:1 filtriert, wobei 165 mg 22-Hydroxi-1-docosanthiol erhalten wurden. DC(Toluol : EtOAc = 2:1) Rf-Werte von Produkt und Edukt sind sehr ähnlich, das Edukt zeigt aber Fluoreszenzlöschung(Aromat).

Das als Ausgangsmaterial verwendete 22-Tosyloxi-1-docosanol wurde wie folgt hergestellt:
Eine Lösung von 25,1 g (0,1Mol) 11-Bromundecanol in 60ml trockenem Pyridin wurde unter Rühren tropfenweise mit 20 g (0,12Mol) Bis-trimethylsilylacetamid versetzt. Das Reaktionsgemisch wurde nach beendetem Zutropfen noch 2h bei Raumtemp. gerührt. Dann wurde mit tert. Butylmethylether und Wasser aufgenommen, die Etherphase dreimal mit Wasser gewaschen, über Natriumsulfat getrocknet und am Rollverdampfer eingeengt. Der Rückstand wurde mit Toluol aufgenommen und die Lösung durch Kieselgel filtriert. Nach Entfernen des Toluols wurde 11-Brom-1-undecyltrimethylsilylether in praktisch quantitativer Ausbeute erhalten. DC (Toluol) Rf=0,8.

0,33 g (15mMol) metallisches Natrium wurden in Toluol auf 120° unter Rühren erwärmt zur feinen Verteilung. Nach Abkühlung auf Raumtemp. wurden 4,85 g (15mMol)11-Brom-undecyltrimethylsilylether unter Rühren zugetropft und das Gemisch wieder auf 120° erhitzt, wobei allmählich Natriumbromid ausfällt und die Lösung sich blau färbt. Nach 1h wurde Lithiumtetrachlorocuprat als Kopplungskatalysator zugefügt und das Gemisch weitere 2h am Rückfluss erhitzt. Nach Abkühlen auf Raumtemp. wurde mit 50ml tert.Butylmethylether versetzt, und Reste Natrium wurden durch tropfenweise Zugabe von Wasser zersetzt. Die organische Phase wurde an Kieselgel filtriert und am Rotationsverdampfer eingeengt. (2,8 g = 77%) DC (Toluol) Rf=0,75 DC(Toluol:EtOAc=2:1)Rf=0,9.

Das voranstehend erhaltene 1,22-Di-(Trimethylsilyloxi)docosan wurde in 30ml tert. Butylmethylether gelöst und die Lösung unter Rühren mit 20 ml 1n HCl versetzt. Es tritt rasch Abscheidung von kristallinem 1,22-Dihydroxidocosan ein. Nach 1h Rühren wurde das ausgefallene Material abgetrennt und am HV getrocknet. Ausbeute praktisch quantitativ (ca.1,9g) DC (Toluol:EtOAc=2:1) Rf = 0,25.

230 mg (0,67mMol)1,22-Dihydroxidocosan in 5 ml Pyridin wurden unter Rühren tropfenweise mit 127 mg (0,67mMol) Tosylchlorid in 1ml Pyridin versetzt und die Mischung anschliessend während 2,5h bei Raumtemp. gerührt. Dann wurde das Reaktionsgemisch mit 50ml Toluol in einen Scheidetrichter transferiert und mit 30ml Wasser geschüttelt. Nach Abtrennen der Wasserphase wurde die organische Phase mit 2n Schwefelsäure (Entfernung von Pyridin), anschliessend mit Hydrogencarbonat-Lösung gewaschen und nach dem Trocknen über Natriumsulfat am Rollverdampfer eingedampft, wobei ein weisses Pulver erhalten wurde. Durch Chromatographie an Kieselgel mit Toluol: EtOAc= 2:1 wurde nicht umgesetztes Diol und Spuren Ditosylat entfernt.Es wurden 74 mg reines 22-Tosyloxi-1-docosanol erhalten. DC (Toluol:EtOAc= 2:1) Rf=0,55.

Beispiel 2: 50mg(0,1mMol)22-Tosyloxi-1-docosanol wurden zusammen mit 2,5ml(0,05mMol) einer 0,02M ethanolischen Lösung von Natriumsulfid 2h am Rückfluss erhitzt. Dann wurde mit Wasser/Chloroform aufgenommen, die organische Phase abgetrennt und am Rollverdampfer eingeengt. Nach Chromatographie des Rückstandes an Kieselgel mit Toluol:EtOAc=2:1 wurden 10mg Di-(22-Hydroxidocosyl)-sulfid rein erhalten.

Beispiel 3: Eine Lösung von 230mg(0,6mMol) 4-(3'-Hydroxi-bromtridecanyl)-2,2-dimethyl-1,3-dioxolan und 140mg(1,2mMol) Kaliumthioacetat in 4ml Ethanol wurde während 2h am Rückfluss erwärmt. Dann wurde am Rollverdampfer eingeengt und der Rückstand an Kieselgel mit Toluol: EtOAc=2:1 chromatographiert und dabei 194mg 4-(3'-Hydroxi-13'-thioacetoxitridecanyl)-2,2-dimethyl-1,3-dioxolan isoliert.

Nach Lösen von 4-(3'-Hydroxi-13'- thioacetoxitridecanyl)-2,2-dimethyl-1,3-dioxolan in 5ml Methanol wurden 0,5ml 1n KOH zugefügt und über Nacht bei Raumtemp. gerührt (Verseifung Thioacetat), anschliessend wurde die Lösung mit 1,5ml Ionenaustauscher Dowex 50W X8(H+) während 3h gerührt und zur Spaltung des Acetonides noch auf 45° erwärmt (Umacetalisierung). Nach Entfernen des Methanols am Rollverdampfer wurde der Rückstand an Kieselgel chromatographiert. Man erhielt 90mg(51%) 11-14-15-Trihydroxi-1-pentadecanthiol. DC(RP8;3% Chloroform/Ethanol)Rf=0,4.

Das als Ausgangsmaterial eingesetzte 4-(3'-Hydroxi-bromtridecanyl)-2,2-dimethyl-1,3-dioxolan wurde wie folgt hergestellt: Eine Lösung von 2,1g(10 mMol)4-Bromethyl-2,2-dimethyl-1,3-dioxolan in 25ml Ether wurde in üblicher Weise durch Umsetzung mit Magnesium in das Grignard-Reagenz übergeführt. Die erhaltene Etherlösung wurde auf 0° abgekühlt und tropfenweise mit einer Lösung von 1g (6,25mMol) 11-Brom-1-undecanal in 5ml Ether unter Rühren versetzt. Nach Rühren bei Raumtemp. über Nacht wurde Natriumbicarbonatlösung zugegeben und das Reaktionsprodukt mit Ether extrahiert. Die organische Phase wurde über Natriumsulfat getrocknet und am Rollverdampfer eingeengt, der Rückstand an Kieselgel mit Toluol:EtOAc=2:1 chromatographiert und 1,7g(45%) 4-(3'-Hydroxi-13'-bromtridecanyl)-2,2-dimethyl-1,3-dioxolan erhalten.

Beispiel 4: In Analogie zu Literatur wurde p-Azidobenzoylchlorid aus 0,652g (4mMol) der entsprechenden Säure in Thionylchlorid hergestellt. Das nach Einengen erhaltene rohe Säurechlorid wurde zugegeben zu einer Suspension von 0,994g(2mMol)Di(11-thioundecyl)disufid dinatriumsalz und über Nacht bei Raumtemp. nachgerührt. Dann wurde bei 70° im WSV eingeengt und der Rückstand mit Toluol/conc.HCl aufgenommen, an Kieselgel filtriert und mit Toluol:EtOAc=2:1 nachgewaschen.Das Eluat, welches die Reinfraktion enthielt, wurde eingeengt und ergab 1,15g (79%) Di(11-(p-Azidobenzoylmerkapto)-undecyl)disulfid (Rf-Wert= 0,1)welches im NMR und Elementaranalyse charakterisiert wurde.

Das als Ausgangsmaterial verwendete Di(11thioundecyl)disufiddinatriumsalz wurde wie folgt hergestellt: Eine Lösung von 6,56g (20 mMol) 1,11-Dibromundecan in 200ml Ethanol wurde versetzt mit 20,8g(200 mMol) Kaliumthioacetat. Es wurde 16h rückflussiert. Die entstandene Lösung wurde im WSV eingeengt, anschliessend mit Toluol aufgenommen, an Kieselgel filtriert und mit Toluol nachgewaschen. Das Eluat wurde eingeengt und ergab 6,32g(99%) 1,11 -Diacetylmerkaptoundecan als gelbliches Oel welches im MS charakterisiert wurde.

Es wurden 3,185g(10 mMol)1,11-Diacetylmerkaptoundecan gelöst in 100ml Ethanol(es enstand bei Rühren erst eine Lösung, welche nach1h kristallisierte). Die Suspension wurde anschliessend 16h bei Raumtemp. gerührt. Dann wurde filtriert, der Rückstand mit Ethanol gewaschen und getrocknet.Man erhiehlt 0,88g(33%)1,11-Undecandithiol-dinatriumsalz vom Schmelzpunkt = 61-62° mit korrekter Elementaranalyse Das Filtrat wurde mit 200ml Wasser versetzt und anschliessend filtriert. Der Rückstand wurde mit Wasser gewaschen und getrocknet und ergab 1,36g(54%)Di(11-thioundecyl)disulfid-dinatriumslz vom Schmelzpunkt = 292-294°welches im MS und Elementaranalyse charakterisiert wurde.

Beispiel 5: Man rückflussierte 3d 100mg 11-Phthalimidoundecylthioacetat in 5ml Hydrazin. Dann wurde die Lösung eingeengt und mit siedender Salzsäure(conc.) gelöst und wieder eingeengt. Das weisse kristalline Rohprodukt wurde aufgenommen in Methanol und an silanisiertem Kieselgel chromatographiert. Das so erhaltene 11-Aminoundecylthiol als Hydrochlorid wurde im MS und NMR charakterisiert. Die Elementaranalyse ergab, dass das Produkt aus 19 Mol% der gewünschten Verbindung und 81 Mol% Hydrazinium monohydrochlorid bestand.

Das als Ausgangsmaterial eingesetzte 11-Phthalimidoundecylthioacetat wurde wie folgt hergestellt:
Eine Lösung von 12,56 g (50 mMol) 11-Bromundecanol in 100 ml DMF wurden versetzt mit 18,52 g (100 mMol)Phthalimid-Kalium und 16 h am Rückfluss gekocht. Dann wurde im WSV eingeengt, mit Toluol : EtOAc=2:1 chromatographiert (Rf=0,5) und ergab 15,6 g (98%)11-Phthalimino-undecanol vom Schmelzpunkt = 86-87°, welches im NMR kontrolliert wurde.HPLC(30% MeCN; RP18 125-4) tR=3,5 min.

Eine Lösung von 4,76 g (15 mMol) 11-Phthalimidoundecanol und 4,34 g (18m Mol) Tosylchlorid in 50 ml Pyridin wurden 2h bei Raumtemp. gerührt. Dann wurde 1 h mit gesättigter Natriumbicarbonat-Lösung gerührt und anschliessend mit Methylenchlorid extrahiert. Die organischen Phasen wurden mit Wasser, dann mit 1n Salzsäure und nochmals mit Wasser gewaschen. Es wurde über Natriumsulfat getrocknet und eingeengt. Man erhielt 5,9 g (85%) 11-Phthalimido-undecyltosylat, welches im NMR und HPLC kontrolliert wurde (tR=12 min.). DC(Toluol)Rf=0,08
Es wurden 3g (6,36 mMol) 11-Phthalimidoundecanyltosylat gelöst in 150ml Ethanol und mit 1,45 g (12,7 mMol) Kaliumthioacetat 16 h gekocht. Dann wurde eingeengt und anschliessend mit Toluol aufgenommen. Die Suspension wurde an Kieselgel chromatographiert. Das HV-getrocknete 11-Phthalimidoundecylthioacetat wurde mittels NMR charakterisiert. DC (Toluol) Rf=0,14.

Beispiel 6: Immobilisierung von IgG auf der Goldoberfläche eines SPR-Sensors via Aldehydfunktionen an Zuckerresten.

Für dieses Immobilisierungsverfahren werden an den Zuckerresten der IgG-Moleküle durch Oxidation freie Aldehydfunktionen erzeugt, welche dann mit Hydrazidfunktionen auf der Goldoberfläche reagieren.

Zur Herstellung einer für die Immobilisierung geeigneten hydrazidhaltigen organischen Zusatzschicht( 30% Hydrazid-, 50% Hydroxylfunktionen) auf einer Goldoberfläche wird die Goldoberfläche während 12 h mit einer methanolischen Lösung von Mercaptoundecansäure (1*10⁻³ mol/l) und Mercaptoundecanol (1*10⁻³ mol/l) in Kontakt gebracht. Bei dieser Behandlung bildet sich spontan eine monomolekulare Schicht aus. Die in dieser monomolekularen Schicht vorhandenen Carbonsäurefunktionen werden mit Chlorameisensäureethylester aktiviert und in Säurehydrazidfunktionen umgewandelt. Die Aktivierung erfolgt durch Eintauchen der Oberfläche in eine Lösung von Chlorameisensäureethylester (5% v/v) in Methylenchlorid und Pyridin (4% v/v) unter Innertgasatmosphäre während 1h. Die Umwandlung der aktivierten Carbonsäurefunktionen zu Hydraziden erfolgt durch Umsetzen der Oberfläche mit einer Lösung von Hydrazin(ca. 1% v/v) in Methylenchlorid. Diese Oberfläche kann dann mit den für dieses Immobilisierungsverfahren modifizierten Antikörpern zur Reaktion gebracht werden.

Die Modifikation der IgG-Moleküle erfolgt nach an sich bekannter Weise durch eine Behandlung der Proteine mit Natriumperjodat. Die Antikörper werden in Acetatpuffer (0.1 M; pH 5.5) gelöst (20 mg/ml), und dazu wird von einer 0.5 M Natriumperjodat-Lösung soviel zugegeben, dass eine Konzentration von 10mM vorliegt. Die Reaktion dauert 20 min. bei 0°C.

Für die Immobilisierung der so modifizierten Antikörper wird diese Reaktionslösung während 1 h mit obiger Hydrazidoberfläche in Kontakt gebracht.

Immobilisierung von Fab'-Fragmenten auf der Goldoberfläche eines SPR-Sensors via die SH-Funktionen freier Cysteine.

Für die Immobilisierung werden die Fab'-Fragmente nach an sich bekannten Verfahren durch Oxidation der Disulfidbrücken aus Fab'-Fragmenten von Antikörpern erzeugt. Letztere gewinnt man auf bekannte Art und Weise durch eine enzymatische Spaltung der Antikörper.

Zur Herstellung einer für die Immobilisierung der Fab'-Fragmente geeigneten Zusatzschicht auf Goldoberflächen wird die Goldoberfläche während 48 Stunden mit einer methanolischen Lösung von 11-Aminoundekanamin (1*10⁻³mol/l) in Kontakt gebracht. Um SH-spezifische, reaktive Gruppen in die Oberfläche einzubringen werden die Aminogruppen mit N-succinimidy-3(2-pyridinyl)-dithiopropionat (Fluka) (1*10⁻³mol/l in Isobutanol) umgesetzt. Dieser Oberfläche kann dann mit den für die Immobilisierung hergestellten Fab'-Fragmenten eines Antikörpers zur Reaktion gebracht werden.

## Patentansprüche

1. Immunosensorischer Wandler bestehend aus einem Träger aus Gold, einer auf der Träger-Oberfläche über schwefelhaltige Gruppen angelagerten monomolekularen organischen Zwischenschicht aus Alkylderivaten und einer auf dieser Zwischenschicht immobilisierten Liganden- oder Antiligandenschicht, dadurch gekennzeichnet, dass die Alkylderivate in ω-Stellung zur schwefelhaltigen Gruppe funktionelle Gruppen tragen und die organische Zwischenschicht aus hydroxylhaltigen Alkylderivaten besteht, welche die spontane Adsorption der Proteine während einer Immobilisierung verhindern, sowie aus Alkylderivaten, welche über spezielle Gruppen für das kovalente Binden von Proteinen verfügen.

2. Immunosensorischer Wandler nach Anspruch 1, dadurch gekennzeichnet, dass die hydroxylhaltigen Alkylderivate einen Hydoxyl-Rest, einen Polyalkohol-Rest, einen Ethylenglykol-Rest oder einen Monosaccharid-Rest tragen.

3. Immunosensorischer Wandler nach Anspruch 1, dadurch gekennzeichnet, dass die speziellen Gruppen für das kovalente Binden von Proteinen einen für die Proteinimmobilisierung aktivierbaren Rest wie Hydroxyl, Carboxyl, Aminophenyl, Nitrophenyl oder Phenylazido tragen.

4. Immunosensorischer Wandler nach Anspruch 3, dadurch gekennzeichnet, dass die speziellen Gruppen für das kovalente Binden von Proteinen ein Rest wie Cyanat, Oxycarbonylimidazol, 2-Oxychinon, 2-Oxymethylenchinon, -Oxy(dichlortriazin), 1-Oxy-2,3-epoxypropan, (2-Oxyethyl)-vinylsulfon, (2,2,2-Trifluorethyl)-sulfonyloxy, (p-Tolyl)-sulfonyloxy, p-(Oxybenzoyl)-nitren, p-(Mercaptobenzoyl)-nitren, p-(Oxybenzyl)-diazoniumchlorid, Pyridyldithiopropionsäureester, gemischtes Anhydrid, Isoharnstoffester, Hydroxysuccinimidester, Säurechlorid, Säurehydrazid, Säureazid, 2-Acylaminoethylpyridyldisulphid, Maleinimid, p-(Aminobenzoyl)-nitren, Pyridyldithio-propionamid oder ein Isocyanat ist, und ohne zusätzliche Aktivierung für die Immobilisierung von Proteinen verwendet werden kann.

5. Verfahren zur Herstellung eines Wandlers nach einem der vorangehenden Ansprüche, dadurch gekennzeichnet, dass auf einer Oberfläche eines Trägers aus Gold durch spontane Chemisorption eine monomolekulare Schicht aus einem Gemisch von Alkylderivaten mit schwefelhaltigen Gruppen aufgebracht wir, wobei die Alkylderivate in ω-Stellung zur schwefelhaltigen Gruppe funktionelle Gruppen tragen und das Gemisch aus hydroxylhaltigen Alkylderivaten besteht, welche die spontane Adsorption der Proteine während einer Immobilisierung verhindern, sowie aus Alkylderivaten, welche über spezielle Gruppen für das kovalente Binden von Proteinen verfügen, und anschliessend auf dieser Schicht ein für den gewünschten Immunnachweis geeigneter Ligand oder Antiligand immobilisiert wird.

## Claims

1. An immunosensory transducer comprising a gold carrier, a monomolecular organic intermediate layer of alkyl derivatives arranged on the carrier surface via sulphur-containing groups and a ligand or antiligand layer immobilized on this intermediate layer, characterized in that the alkyl derivatives carry functional groups in the ω-position to the sulphur-containing groups and the organic intermediate layer consists of hydroxyl-containing alkyl derivatives which prevent the spontaneous adsorption of proteins during an immobilization and of alkyl derivatives which have special groups for the covalent binding of proteins.

2. An immunosensory transducer according to claim 1, characterized in that the hydroxyl-containing alkyl derivatives carry a hydroxyl residue, a polyalcohol residue, an ethylene glycol residue or a mononsaccharide residue.

3. An immunosensory transducer according to claim 1, characterized in that the special groups for the covalent binding of proteins contain a residue such as hydroxyl, carboxyl, aminophenyl, nitrophenyl or phenylazido which can be activated for the protein immobilization.

4. An immunosensory transducer according to claim 3, characterized in that the special group for the covalent binding of proteins is a residue such as cyanate, oxycarbonylimidazole, 2-oxyquinone, 2-oxymethylenequinone, -oxy(dichlorotriazine), 1-oxy-2,3-epoxypropane, (2-oxyethyl)-vinylsulphone, (2,2,2-trifluoroethyl)-sulphonyloxy, (p-tolyl)-sulphonyloxy, p-(oxybenzolyl)-nitrene, p-mercaptobenzoyl)-nitrene, p-(oxybenzyl)-diazonium chloride, pyridyldithiopropionic acid ester, mixed anhydride, isourea ester, hydroxysuccinimide ester, acid chloride, acid hydrazide, acid azide, 2-acylaminoethyl pyridyl disulphide, maleimide, p-(aminobenzoyl)-nitrene, pyridyldithio-propionamide or an isocyanate and can be used without additional activation for the immobilization of proteins.

5. A method for the production of a transducer according to any one of the preceding claims, characterized by applying a monomolecular layer of a mixture of alkyl derivatives with sulphur-containing groups to a surface of a gold carrier by spontaneous chemisorption, whereby the alkyl derivatives carry functional groups in the ω-position to the sulphur-containing groups which prevent the spontaneous adsorption of proteins during an immobilization and of alkyl derivatives which have special groups for the covalent binding of proteins, and subsequently immobilizing on this layer a ligand or antiligand suitable for the desired immunodetection.

## Revendications

1. Transducteur pour immunocapteur, constitué d'un support à base d'or, d'une couche intermédiaire organique monomoléculaire, à base de dérivés alkyliques, fixée sur la surface du support par l'intermédiaire de groupes soufrés, et d'une couche de ligand ou d'antiligand immobilisé sur cette couche intermédiaire, caractérisé en ce que les dérivés alkyliques portent des groupes fonctionnels en position ω par rapport au groupe soufré, et la couche intermédiaire organique est constituée de dérivés alkyliques hydroxylés qui empêchent l'adsorption spontanée des protéines pendant une immobilisation, ainsi que de dérivés alkyliques qui disposent de groupes spéciaux pour la liaison covalente de protéines.

2. Transducteur pour immunocapteur selon la revendication 1, caractérisé en ce que les dérivés alkyliques hydroxylés portent un radical hydroxy, un radical polyol, un radical éthylèneglycol ou un reste de monosaccharide.

3. Transducteur pour immunocapteur selon la revendication 1, caractérisé en ce qui les groupes spéciaux pour la liaison covalente de protéines portent un radical activable pour l'immobilisation de protéines, tel qu'un radical hydroxy, carboxy, aminophényle, nitrophényle ou phénylazido.

4. Transducteur pour immunocapteur selon la revendication 3, caractérisé en ce que les groupes spéciaux pour la liaison covalente de protéines sont des radicaux tels que les radicaux cyanate, oxycarbonylimidazole, 2-oxyquinone, 2-oxyméthylquinone, oxy(dichlorotriazine), 1-oxy-2,3-époxypropane, (2-oxyéthyl)vinylsulfone, (2,2,2-trifluoroéthyl)sulfonyloxy, (p-tolyl)sulfonyloxy, p-(oxybenzoyl)nitrène, p-(mercaptobenzoyl)nitrène, chlorure de p-(oxybenzyl)diazonium, dithiopropionate de pyridyle, anhydride mixte, ester d'iso-urée, ester d'hydroxysuccinimide, chlorure d'acide, hydrazide d'acide, disulfure de 2-acylaminoéthylpyridyle, maléimide, p-(aminobenzoyl)nitrène, pyridyldithiopropionamide ou un isocyanate, et peuvent être utilisés pour l'immobilisation de protéines sans activation supplémentaire.

5. Procédé pour la fabrication d'un transducteur selon l'une des revendications précédentes, caractérisé en ce que l'on applique sur une surface d'un support à base d'or, par chimiosorption spontanée, une couche monomoléculaire d'un mélange de dérivés alkyliques à groupes soufrés, les dérivés alkyliques portant des groupes fonctionnels en position ω par rapport au groupe soufré, et le mélange étant constitué de dérivés alkyliques hydroxylés qui empêchent l'adsorption spontanée des protéines pendant une immobilisation, ainsi que de dérivés alkyliques qui disposent de groupes spéciaux pour la liaison covalente de protéines, et un ligand ou antiligand approprié à l'immunodétection désirée est ensuite immobilisé sur cette couche.
